(19) Europäisches Patentamt
European Patent Office
Office européen des brevets

(11) Veröffentlichungsnummer: **0 229 383**
**B1**

(12) # EUROPÄISCHE PATENTSCHRIFT

(45) Veröffentlichungstag der Patentschrift: **29.08.90**

(51) Int. Cl.[5]: **C 07 D 211/90**, C 07 B 59/00, G 01 N 33/534

(21) Anmeldenummer: **86117975.2**

(22) Anmeldetag: **23.12.86**

(54) **35s Markierte 1,4-Dihydropyridine, Verfahren zur Herstellung und ihre Verwendung.**

(30) Priorität: **11.01.86 DE 3600593**

(43) Veröffentlichungstag der Anmeldung:
**22.07.87 Patentblatt 87/30**

(45) Bekanntmachung des Hinweises auf die Patenterteilung:
**29.08.90 Patentblatt 90/35**

(84) Benannte Vertragsstaaten:
**AT BE CH DE ES FR GB IT LI NL SE**

(56) Entgegenhaltungen:
**EP-A-0 181 061**
**DE-A-3 341 806**

**ULLMANNS "Encyklopädie der technischen Chemie", 4. Auflage, Band 20, 1981, Seiten 66,67, Verlag Chemie, Weinheim**

**Die Akte enthält technische Angaben, die nach dem Eingang der Anmeldung eingereicht wurden und die nicht in dieser Patentschrift enthalten sind.**

(73) Patentinhaber: **BAYER AG**
**D-5090 Leverkusen 1 Bayerwerk (DE)**

(72) Erfinder: **Glossmann, Hartmut, Prof.Dr.**
**Margaritenweg 4**
**D-6301 Heuchelheim (DE)**

**EP 0 229 383 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Die Erfindung betrifft radioaktiv mit $^{35}S$ markierte 1,4-Dihydropyridine, Verfahren zu ihrer Herstellung und ihre Verwendung zur Ausprüfung von Arzneimitteln oder anderen Stoffen mit Radioreceptorassays sowie zur Blutplasmaspiegelbestimmung von Dihydropyridinen.

Unter Calciumantagonisten versteht man Stoffe, die den Durchtritt von Calcium durch die Calciumkanäle der Zellmembran blockieren. Sie werden unter anderem in folgenden Gebieten therapeutisch angewendet: Angina pectoris, Herzrhythmusstörungen, Bluthochdruck, Vasospasmus [D. J. Triggle, Calcium antagonists — Basic Chemical and Pharmacological Aspects S. 1—18 in G. B. Weiss "New Perspectives on Calcium Antagonists", American Physiological Society, Bethesda, Maryland USA (1981)].

Calciumantagonisten gehören verschiedenen chemischen Stoffklassen an, wobei die Vertreter der 1,4-Dihydropyridine mit zu den wirksamsten gehören, da sie schon bei geringen Konzentrationen den Durchtritt von Calcium durch die Calciumkanäle der Zellmembran der glatten Muskulator blockieren. Diese Blockade ist im Fall chiraler 1,4-Dihydropyridine stereoselektiv [A. Fleckenstein (1983), R. A. Janis and D. J. Triggle (1983), R. Towart, E. Wehinger, H. Meyer "Effects of unsymmetrical ester substituted 1,4-dihydropyridine derivatives and their optical isomeres on contraction of smooth muscle, Naunyn-Schmiedeberg's Arch. Pharmacol. *317*, 183—5 (1981)].

Zur Charakterisierung von Bindungsstellen dieser Pharmaka (Rezeptoren) wurde hierbei die Verwendung von radioaktiv mit Tritium oder $^{125}I$ markierten Dihydropyridinen beschrieben [H. Glossmann, D. R. Ferry, F. Lübbecke, R. Mews, F. Hoffmann, "Calcium channels-Direct identification with radioligand binding studies", TIPS *3*, 431—7 (1982) DE—OS 33 41 806].

Ferner wurden mit Tritium oder $^{125}$Iod markierte 1,4-Dihydropyridine zur Blutplasmaspiegelbestimmung von Calciumantagonisten sowie zur autoradiographischen Darstellung ihrer Rezeptoren eingesetzt [R. Quirin, "Autoradiographic localisation of a calcium channel antagonist-3H-nitrendipine, bindung site in rat brain", Neuroscience Lett, *36*, 267—71 (1983), DE—OS 33 41 806].

In Anbetracht der zur Zeit geltenden Bestimmung zur Beseitigung von radioaktiven Abfällen sind jedoch $^{125}I$- sowie vor allem $^{3}H$-haltige Stoffe nicht unbedenklich. Ebenso besitzen die bisher bekannten radioaktiv markierten 1,4-Dihydropyridine oft nur unzureichende Affinität und Spezifität für die entsprechenden Rezeptoren.

Es war daher notwendig, radioaktiv markierte 1,4-Dihydropyridine zu finden, die zur Ausprüfung von Arzneimitteln mit Radiorezeptorassays geeignet sind, eine hohe spezifische sowie radiochemische Stabilität haben, einfach herzustellen sind, eine hinreichend kurze Halbwertzeit haben, unproblematisch zu entsorgen sind und die vor allem hohe Affinitäten und Spezifitäten für die 1,4-Dihdyropyridin-Rezeptoren besitzen.

Die vorliegende Erfindung betrifft nun radioaktiv mit $^{35}S$-markierte 1,4-Dihydropyridine der allgemeinen Formel (I),

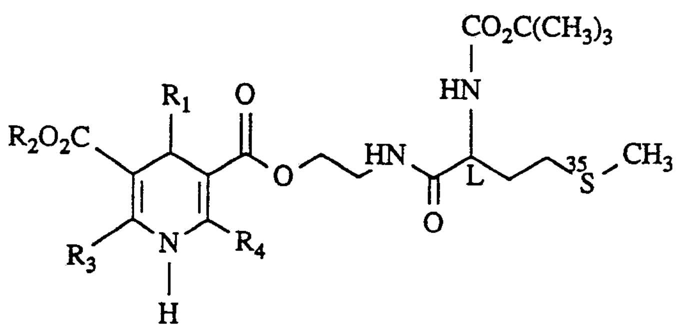

(I)

in welcher

$R_1$ für 2-Nitrophenyl, 3-Nitrophenyl, 2,3-Dichlorphenyl, 2-Chlorphenyl, 2-Trifluormethylphenyl, 2-Difluormethoxyphenyl oder Benzoxadiazolyl steht,

$R_2$ für geradkettiges oder verzweigtes Alkyl mit bis zu 6 Kohlenstoffatomen steht, das gegebenenfalls durch Fluor, Chlor, Brom, N-Benzyl-N-methylamino oder durch Methoxy substituiert ist,

und

$R_3$, $R_4$ gleich oder verschieden sind und jeweils für geradkettiges oder verzweigtes Alkyl mit bis zu 4 Kohlenstoffatomen stehen, oder

für Hydroxymethyl stehen.

Besonders bevorzugt sind Verbindungen der allgemeinen Formel (I), in welcher

$R^1$ für 2-Nitrophenyl, 3-Nitrophenyl, 2,3-Dichlorphenyl oder 2-Trifluormethylphenyl steht,

$R^2$ für geradkettiges oder verzweigtes Alkyl mit bis zu 6 Kohlenstoffatomen steht, das gegebenenfalls durch Methoxy substituiert ist,

und

$R^3$, $R^4$ für Methyl stehen.

Je nach Art der Substituenten können die erfindungsgemäßen Verbindungen in stereoisomeren Formen existieren, die sich nicht wie Bild und Spiegelbild (Diastereomere) verhalten. Sowohl die Diastereomerengemische als die Racemformen sind Gegenstand der Erfindung.

Die erfindungsgemäßen Verbindungen werden hergestellt, indem man einen molaren Überschuß eines Dihydropyridinderivates der allgemeinen Formel (II)

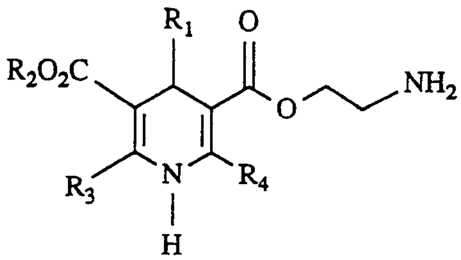

(II)

in welcher

$R^1$—$R^4$ die oben angegebene Bedeutung haben,

mit t-Butoxycarbonyl-L-$^{35}$S-Methionin-N-Succinimidylester der Formel (III)

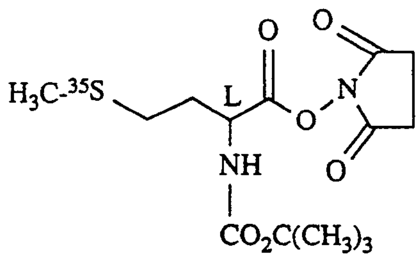

(III)

in inerten organischen Lösungsmitteln in einem pH-Bereich von ca. 7 bis 11 und bei Temperaturen von ca. −30°C bis +80°C umsetzt.

Die Reaktion kann durch folgende Reaktionsgleichung verdeutlicht werden:

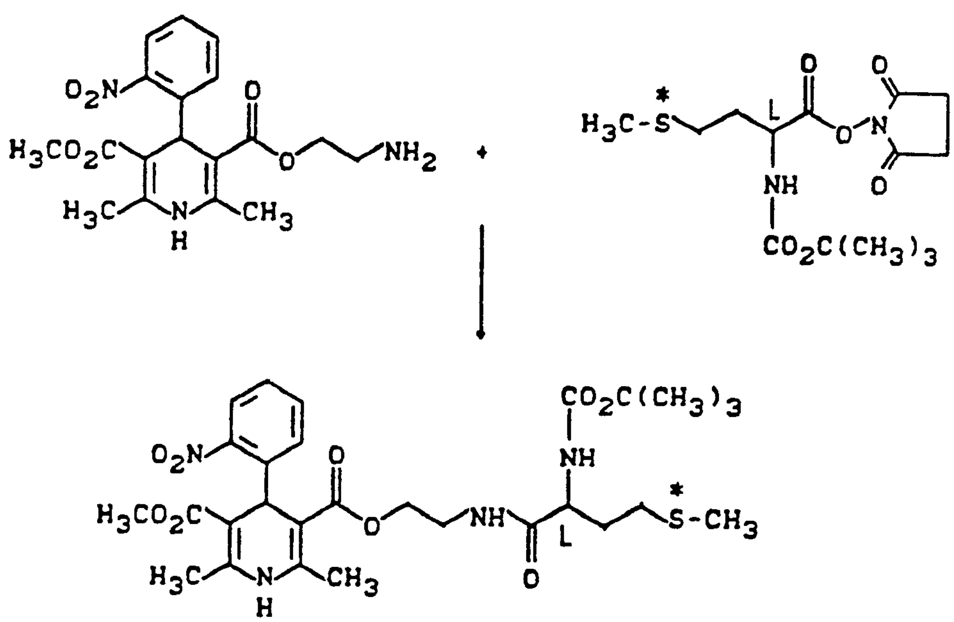

Als Lösungsmittel eignen sich die üblichen inerten organischen Lösungsmittel, die sich unter den Reaktionsbedingungen nicht verändern. Hierzu gehören bevorzugt Alkohole wie Methanol, Ethanol, Propanol oder Isopropanol, oder Ether wie Diethylether, Dioxan, Tetrahydrofuran oder DMF, Hexamethylphosphorsäuretriamid oder Halogenwasserstoff wie beispielsweise Methylenchlorid, Chloroform oder Tetrachlorkohlenstoff, oder Acetonitril oder Essigester. Ebenso ist es möglich, Gemische der genannten Lösungsmittel zu verwenden.

Die Reaktion wird in einem Temperaturbereich von −30°C bis +80°C, bevorzugt von −20°C bis +40°C, durchgeführt.

Die Umsetzung kann bei normalem, aber auch bei erhöhtem oder erniedrigtem Druck erfolgen. Im allgemeinen arbeitet man bei Normaldruck.

Es hat sich bei der Durchführung der Reaktion als günstig erwiesen, in einem bestimmten pH-Bereich zu arbeiten. Im allgemeinen wird die Reaktion in einem pH-Bereioch von 7 bis 11 durchgeführt. Gegebenenfalls wird hierfür eine Base wie Triethylamin oder ein Puffergemische wie z.B. Boratpuffer oder Phosphatpuffer zugesetzt.

Bei der Durchführung der Umsetzung werden im allgemeinen 1 bis 100, bevorzugt 1 bis 50 Mol Dihydropyridin auf 1 Mol tert.-Butoxycarbonyl-L-$^{35}$S-Methionin-N-Succinimidylester eingesetzt.

Die als Ausgangsstoffe verwendeten Dihydropyridine der allgemeinen Formel (II) sind bekannt oder können nach bekannten Methoden hergestellt werden [US—PS 3 985 758; EP—AS 151 006].

Der als Ausgangsprodukt verwendete radioaktiv mit $^{35}$S-markierte tert.-Butoxycarbonyl-L-$^{35}$S-Methionin-N-Succinimidylester der Formel (III) ist bekannt (käuflich).

Die erfindungsgemäßen $^{35}$S-markierten 1,4-Dihdropyridine eignen sich besonders gut zur Arzneimittelausprüfung durch in-vitro- und in-vivo-Tests, zur autoradiographischen Darstellung der entsprechenden Dihydropyridinrezeptoren in bestimmten Organen sowie zur Blutplasmaspiegelbestimmung von Dihydropyridinen.

Die Einführung des Radioisotops $^{35}$S hat folgende Vorteile: $^{35}$S ist ein reiner β-Emitter mit einer maximalen Energie von 0.167 MeV. Die Halbwertzeit beträgt 87.4 Tage und ist damit wesentlich kürzer als die des $^{3}$H. Bei einer spezifischen Radioaktivität von 1300—1500 Ci/mmol haben die erfindungsgemäßen Verbindungen eine überraschend hohe Affinität zu den 1,4-Dihydropyridin-Rezeptoren, z.B. im Zentralen Nervensystem (ZNS), in Gefäßen, in Skelettmuskeln und im Coronarbereich. So hat die Verbindung des Beispiel 1 eine Dissoziationsgeschwindigkeitskonstante (bei 37°C) von 0.070 ± 0.01 $min^{-1}$ am Skelettmuskel, und von 0.02 $min^{-1}$ (37°C) für den Hirnrezeptor. Die Dissoziationskonstante bei 37°C für den Skelettmuskelrezeptor liegt mit 7 ± 3 pMolar etwa 100 mal niedriger als für Nimodipin.

Die radioaktiv mit $^{35}$S-markierten Verbindungen erlauben außerdem bei hoher Zähleffizienz eine rasche und extrem hochauflösende autoradiographische Darstellung der 1,4-Dihydropyridinrezeptoren.

Die Applikation der erfindungsgemäßen markierten Verbindungen erfolgt nach üblichen Methoden z.B. enteral oder parenteral, insbesondere oral und intravenös.

Die erfindungsgemäßen Verbindungen werden bevorzugt verwendet zur Untersuchung der Pharmakokinetik und der Pharmakodynamik von Dihydropyridinen, insbesondere zur Bestimmung von Metabolisierungen, Ausscheidungsarten und organspezifischen Wirkungen.

Beispiel 1

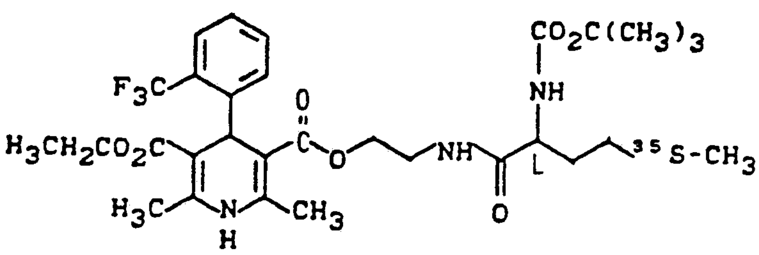

0,5 mCi tert.-Butoxycarbonyl-L-$^{35}$S-Methionin-N-Succinimidylester (1450 Ci/mmol entsprechend 0.34 mMol) werden mit 10 nMol 1,4-Dihydro-2,6-dimethyl-4-(2-trifluormethylphenyl)-pyridin-3,5-dicarbonsäure-3-(2-aminoethyl)-5-ethylester in 10 µl Boratpuffer pH 8.4 (0.1 M) und 10 µl Ethanol für 2 h unter Stickstoff im Dunkeln bei 0°C versetzt. Die Produkte werden auf Kieselgel mit Elution auf Dünnschichtchromatographie bzw. HPLC aufgetrennt und das gewünschte Derivat in radiochemisch reiner Form in guter Ausbeute erhalten. Mit dem Fließmittel Essigsäureethylester — Diethylether (30:70) ist der Rf-Wert 0.66 ± 0.1.

**Patentansprüche**

1. Radioaktiv markierte Dihydropyridine der allgemeinen Formel I

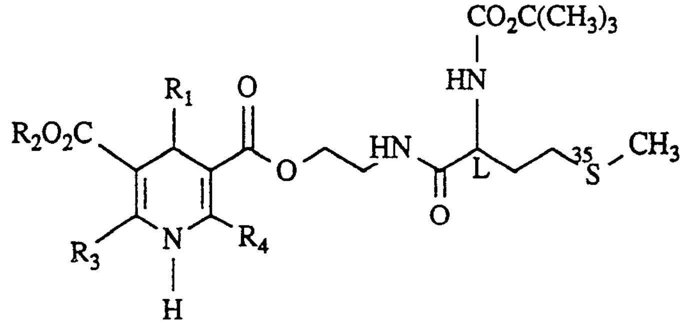

(I)

in welcher

$R_1$ für 2-Nitrophenyl, 3-Nitrophenyl, 2,3-Dichlorphenyl, 2-Chlorphenyl, 2-Trifluormethylphenyl, 2-Difluormethoxyphenyl oder Benzoxadiazolyl steht,

$R_2$ für geradkettiges oder verzweigtes Alkyl mit bis zu 6 Kohlenstoffatomen steht, das gegebenenfalls durch Fluor, Chlor, Brom, N-Benzyl-N-methylamino oder durch Methoxy substituiert ist,

und

$R_3$, $R_4$ gleich oder verschieden sind und jeweils für geradkettiges oder verzweigtes Alkyl mit bis zu 4 Kohlenstoffatomen stehen, oder für Hydroxymethyl stehen.

2. Verbindung der allgemeinen Formel (I) gemäß Anspruch 1, in welcher

$R_1$ für 2-Nitrophenyl, 3-Nitrophenyl, 2,3-Dichlorphenyl oder 2-Trifluormethylphenyl steht,

$R_2$ für geradkettiges oder verzweigtes Alkyl mit bis zu 6 Kohlenstoffatomen steht, das gegebenenfalls durch Methoxy substituiert ist,

und

$R_3$, $R_4$ für Methyl stehen.

3. Verfahren zur Herstellung von Verbindungen der allgemeinen Formel (I)

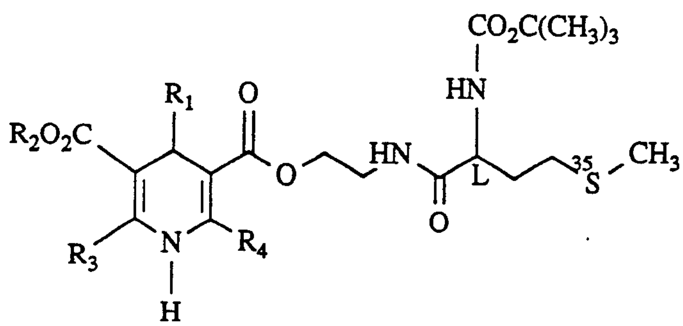

(I)

in welcher

$R_1$ für 2-Nitrophenyl, 3-Nitrophenyl, 2,3-Dichlorphenyl, 2-Chlorphenyl, 2-Trifluormethylphenyl, 2-Difluormethoxyphenyl oder Benzoxadiazolyl steht,

$R_2$ für geradkettiges oder verzweigtes Alkyl mit bis zu 6 Kohlenstoffatomen steht, das gegebenenfalls durch Fluor, Chlor, Brom, N-Benzyl-N-methylamino oder durch Methoxy substituiert ist,

und

$R_3$, $R_4$ gleich oder verschieden sind und jeweils für geradkettiges oder verzweigtes Alkyl mit bis zu 4 Kohlenstoffatomen stehen, oder für Hydroxymethyl stehen,

dadurch gekennzeichnet, daß man einen molaren Überschuß eines Dihydropyridinderivates der allgemeinen Formel II

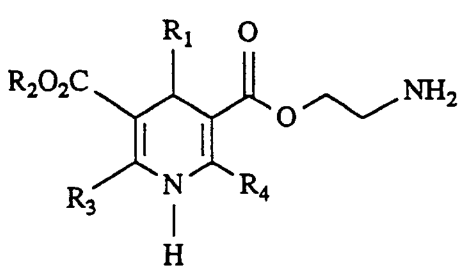

(II)

in welcher

$R_1$—$R_4$ die oben angegebene Bedeutung haben, mit tert.-Butoxycarbonyl-L-$^{35}$S-Methionin-N-Succinimidylester der Formel (III)

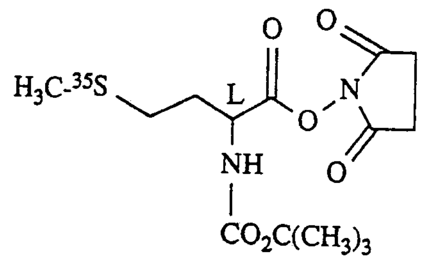

(III)

in inerten organischen Lösungsmitteln in einem pH-Bereich von ca. 7 bis 11 und bei Temperaturen von ca. −30°C bis +80°C umsetzt.

4. Verwendung von Verbindungen der allgemeinen Formel (I) gemäß Anspruch 1 zur Untersuchung der Pharmakokinetik und Pharmakodynamik von Dihydropyridinen.

**Revendications**

1. Dihydropyridines marquées par la radioactivité, de formule générale I

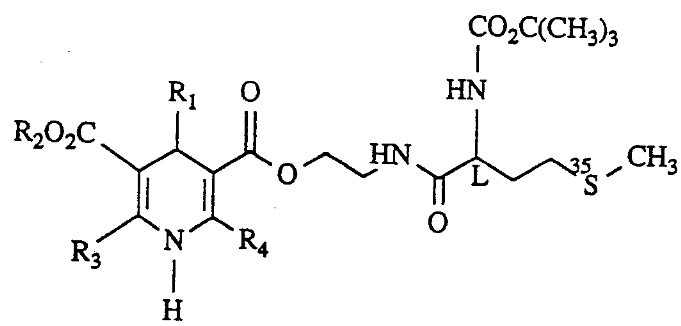

(I)

dans laquelle

$R_1$ est un groupe 2-nitrophényle, 3-nitrophényle, 2,3-dichlorophényle, 2-chlorophényle, 2-trifluorométhylphényle, 2-difluorométhoxyphényle ou benzoxadiazolyle,

$R_2$ est un groupe alkyle à chaîne droite ou ramifiée ayant jusqu'à 6 atomes de carbone, qui est substitué le cas échéant par du fluor, du chlore, du brome, un groupe N-benzyl-N-méthylamino ou par un radical méthoxy,

et

$R_3$ et $R_4$ sont identiques ou différents et représentent chacun un groupe alkyle à chaîne droite ou ramifiée ayant jusqu'à 4 atomes de carbone, ou bien un groupe hydroxyméthyle

2. Composés de formule générale (I) suivant la revendication 1, dans laquelle

$R_1$ est un groupe 2-nitrophényle, 3-nitrophényle, 2,3-dichlorophényle ou 2-trifluorométhylphényle,

$R_2$ est un groupe alkyle à chaîne droite ou ramifiée ayant jusqu'à 6 atomes de carbone, qui est substitué le cas échéant par un radical méthoxy, et

$R_3$, $R_4$ sont des groupes méthyle.

3. Procédé de préparation de composés de formule générale (I)

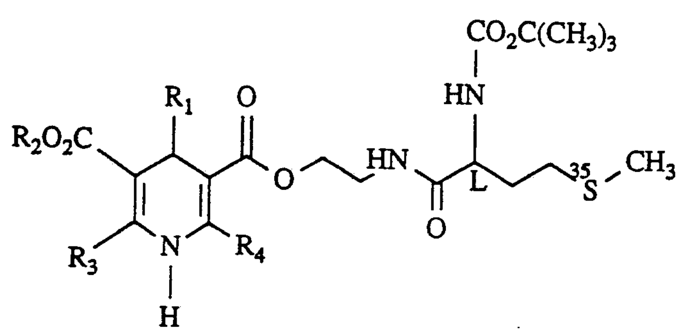

(I)

dans laquelle

$R_1$ est un groupe 2-nitrophényle, 3-nitrophényle, 2,3-dichlorophényle, 2-chlorophényle, 2-trifluorométhylphényle, 2-difluorométhoxyphényle ou benzoxadiazolyle,

$R_2$ est un groupe alkyle à chaîne droite ou ramifiée ayant jusqu'à 6 atomes de carbone, qui est substitué le cas échéant par du fluor, du chlore, du brome, un radical N-benzyl-N-méthylamino ou par un radical méthoxy,

et

$R_3$, $R_4$ sont identiques ou différents et représentent chacun un groupe alkyle à chaîne droite ou à chaîne ramifiée ayant jusqu'à 4 atomes de carbone, ou bien

un groupe hydroxyméthyle, caractérisé en ce qu'on fait réagir un excès molaire d'un dérivé de dihydropyridine de formule générale II

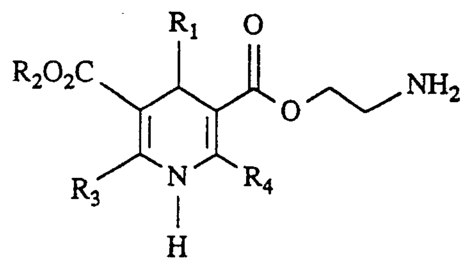

(II)

dans laquelle

$R_1$ à $R_4$ ont la définition indiquée ci-dessus, avec l'ester de N-succinimidyle de tertiobutoxycarbonyl-L-$^{35}$S-méthionine de formule (III)

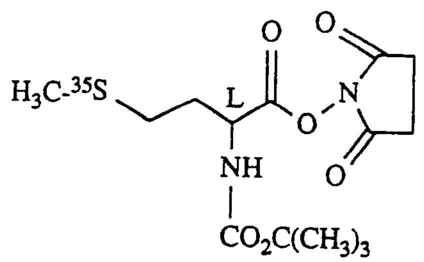

(III)

dans des solvants organiques inertes dans une plage de pH d'environ 7 à 11 et à des températures d'environ −30°C à +80°C.

4. Utilisation de composés de formule générale (I) suivant la revendication 1 pour l'étude de la pharmacocinétique et de la pharmacodynamique de dihydropyridines.

**Claims**

1. Radioactively labelled dihydropyridines of the general formula I

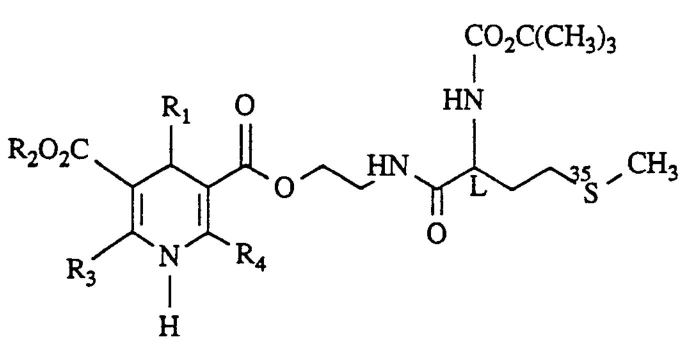

(I)

in which

$R_1$ represents 2-nitrophenyl, 3-nitrophenyl, 2,3-dichlorophenyl, 2-chlorophenyl, 2-trifluoromethylphenyl, 2-difluoromethoxyphenyl or benzoxadiazolyl, $R_2$ repreents straight-chain or branched alkyl which has up to 6 carbon atoms and which is optionally substituted by fluorine, chlorine, bromine, N-benzyl-N-methylamino or by methoxy, and $R_3$ and $R_4$ are identical or different and each represents straight-chain or branched alkyl having up to 4 carbon atoms, or represents hydroxymethyl.

2. Compounds of the general formula (I) according to Claim 1, in which

$R_1$ represents 2-nitrophenyl, 3-nitrophenyl, 2,3-dichlorophenyl or 2-trifluoromethylphenyl, $R_2$ represents straight-chain or branched alkyl which has up to 6 carbon atoms and is optionally substituted by methoxy, and $R_3$ and $R_4$ represent methyl.

3. Process for the preparation of compounds of the general formula (I)

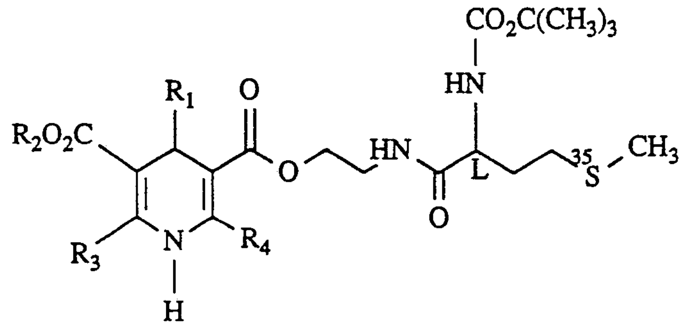

(I)

in which

$R_1$ represents 2-nitrophenyl, 3-nitrophenyl, 2,3-dichlorophenyl, 2-chlorophenyl, 2-trifluoromethylphenyl, 2-difluoromethoxyphenyl or benzoxadiazolyl, $R_2$ represents straight-chain or branched alkyl which has up to 6 carbon atoms and which is optionally substituted by fluorine, chlorine, bromine, N-benzyl-N-methylamino or by methoxy, and $R_3$ and $R_4$ are identical or different and each represents straight-chain or branched alkyl having up to 4 carbon atoms, or represents hydroxymethyl, characterized in that a molar excess of a dihydropyridine derivative of the general formula II

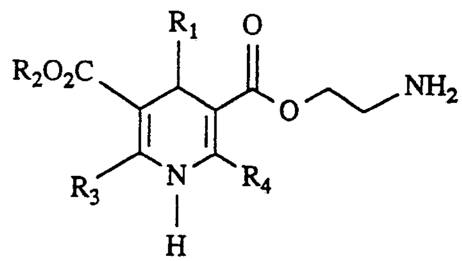

(II)

in which

$R_1$—$R_4$ have the abovementioned meaning, is reacted with tert.-butoxycarbonyl-L-$^{35}$S-methionine N-succinimidyl ester of the formula (III)

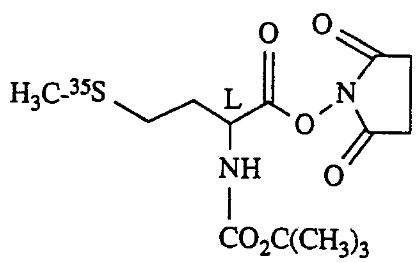

(III)

in inert organic solvents in a pH range from approx 7 to 11 and at temperatures from about −30°C to +80°C.

4. Use of compounds of the general formula (I) according to Claim 1 for investigating the pharmacokinetics and pharmacodynamics of dihydropyridines.